(19)

Europäisches
Patentamt
European
Patent Office
Office européen
des brevets

(11) **EP 4 650 387 A1**

(12) # EUROPEAN PATENT APPLICATION

(43) Date of publication:
**19.11.2025 Bulletin 2025/47**

(21) Application number: **25175535.1**

(22) Date of filing: **09.05.2025**

(51) International Patent Classification (IPC):
***C08J 3/16*** *(2006.01)*  ***A61Q 19/00*** *(2006.01)*
***C08L 1/28*** *(2006.01)*

(52) Cooperative Patent Classification (CPC):
**C08J 3/16; A61K 8/0241; A61K 8/025;
A61K 8/731; A61Q 19/00; C08L 1/284;**
A61K 2800/10; A61K 2800/412; A61K 2800/654;
C08J 2301/28

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **13.05.2024 JP 2024078097**

(71) Applicant: SHIN-ETSU CHEMICAL CO., LTD.,
Chiyoda-ku,
Tokyo 1000005 (JP)

(72) Inventors:
• **KONISHI, Hidekazu**
  **Niigata, 9428601 (JP)**
• **KUSAKI, Fumie**
  **Niigata, 9428601 (JP)**
• **SUDO, Takane**
  **Niigata, 9428601 (JP)**

(74) Representative: **Schulz Junghans**
**Patentanwälte PartGmbB**
**Großbeerenstraße 71**
**10963 Berlin (DE)**

(54) **LOW-SUBSTITUTED CELLULOSE ETHER SPHERICAL MICROPARTICLE, COSMETIC COMPOSITION USING THE SAME, AND METHOD OF PRODUCING SAME**

(57) The objective of the present invention is to provide a low-substituted cellulose ether spherical microparticle that can contribute to improvement of tactile sensation of a cosmetic composition used, regardless of the use or non-use of carbon disulfide. The above objective is solved by a low-substituted cellulose ether spherical microparticle having an average particle size ($D_{50}$) of primary particles on a volume basis by a dry laser diffraction method of 1 $\mu$m to 30 $\mu$m, a sphericity of 0.75 to 1.0, and a surface smoothness of 75% to 100%, and the like.

EP 4 650 387 A1

## Description

Technical Field

The present invention relates to a low-substituted cellulose ether spherical microparticle, a cosmetic composition using the low-substituted cellulose ether spherical microparticle, and a method of producing the low-substituted cellulose spherical microparticle.

Background Art

Spherical microparticles are used in dosage forms such as matting agents, slip agents, and anti-blocking agents in a variety of fields, depending on their particle characteristics. Spherical microparticles are also used in cosmetics for makeup in order to improve the extensibility and other properties of such cosmetics. In recent years, due to environmental issues such as marine pollution caused by microplastics, spherical microparticle constituents added in cosmetics have been being transferred from petroleum-derived synthetic materials to nature-derived natural materials.

As for spherical microparticles made of natural materials, JP H5-200286 A (Patent Document 1) and JP H11-181147 (Patent Document 2) disclose methods of producing a spherical microparticle by coagulating and regenerating a viscose solution in which a natural material cellulose is used as a raw material. In addition, JP 2003-252902 A (Patent Document 3) discloses a method of obtaining a spherical microparticle by spray-drying a dispersion solution composed of low-substituted cellulose ether.

Citation List

Patent Documents

[0004]

　　　　Patent Document 1: JP H5-200286 A
　　　　Patent Document 2: JP H11-181147 A
　　　　Patent Document 3: JP 2003-252902 A

Summary of the Invention

Problems to be Solved by the Invention

However, the methods described in Patent Documents 1 and 2 are undesirable from a standpoint of environmental impact because they use carbon disulfide, which may be an environmental pollutant, to produce the viscose solution.

In the method described in Patent Document 3, the water of the low-substituted cellulose ether dispersion solution is rapidly evaporated by spray-drying, resulting in particle shrinkage. Therefore, it is difficult to obtain a spherical microparticle with higher sphericity and surface smoothness. As a result, the cosmetic composition containing the spherical microparticle obtained by the method according to Patent Document 3 is insufficient in texture and extensibility when applied to the skin, resulting in poor tactile sensation.

In view of the above circumstances, it is an objective of the present invention to provide a low-substituted cellulose ether spherical microparticle that can contribute to an improvement of tactile sensation of a cosmetic composition used, regardless of the use or non-use of carbon disulfide.

Means of Solving the Problems

In earnestly examining to find a way to solve the above-identified problems, the present inventors repeated the trial and error process to find a method to obtain a low-substituted cellulose ether spherical microparticle using environmentally friendly raw materials. As a result, the present inventors succeeded in obtaining a low-substituted cellulose ether spherical microparticle by preparing a W/O type emulsion of low-substituted cellulose ether composed of environmentally friendly raw materials and subjecting the W/O type emulsion to a precipitation coagulation treatment.

Surprisingly, the resulting low-substituted cellulose ether spherical microparticle had a small average particle size of primary particles, and high sphericity and surface smoothness. Because of the properties, the cosmetic composition obtained by using the low-substituted cellulose ether spherical microparticle had good application and moisture retention properties on the skin, and had a pleasant tactile sensation. More surprisingly, such a tactile sensation of the cosmetic composition was achieved even when the low-substituted cellulose ether spherical microparticle was added in a small amount.

Finally, the present inventors succeeded in inventing, as possible means to achieve the objectives of the present invention, a low-substituted cellulose ether spherical microparticle having an average particle size of primary particles, a sphericity, and a surface smoothness in a certain range, and the like. As such, the present invention has been completed on the basis of the findings and successful examples that were first found or obtained by the present inventors.

According to each aspect of the present invention, there are provided the following embodiments of a low-substituted cellulose ether spherical microparticle, a cosmetic composition, and a method of producing a low-substituted cellulose spherical microparticle.

[1] A low-substituted cellulose ether spherical microparticle, having an average particle size ($D_{50}$) of primary particles on a volume basis by a dry laser diffraction method of 1 $\mu$m to 30 $\mu$m, a sphericity of 0.75 to 1.0, and a surface smoothness of 75% to 100%.

[2] The low-substituted cellulose ether spherical microparticle according to the item [1], having a molar substitution of low-substituted cellulose ether of 0.05 to 1.0.

[2A] The low-substituted cellulose ether spherical microparticle according to item [1] or [2], wherein the microparticle consists of a low-substituted cellulose ether selected from low-substituted hydroxypropyl cellulose, low-substituted hydroxyethyl cellulose, low-substituted methyl cellulose, low-substituted hydroxypropylmethyl cellulose, and mixtures of the preceding substituted celluloses.

[2B] The low-substituted cellulose ether spherical microparticle according to item [1] or [2], wherein the microparticle consists of low-substituted hydroxypropyl cellulose.

[2C] The low-substituted cellulose ether spherical microparticle according to item [1] or [2], wherein the microparticle consists of low-substituted hydroxyethyl cellulose.

[2D] The low-substituted cellulose ether spherical microparticle according to item [1] or [2], wherein the microparticle consists of low-substituted methyl cellulose.

[2E] The low-substituted cellulose ether spherical microparticle according to item [1] or [2], wherein the microparticle consists of low-substituted hydroxypropylmethyl cellulose.

[2F] The low-substituted cellulose ether spherical microparticle according to any one of the preceding items, wherein the low-substituted cellulose ether has a molar substitution of 0.05 to 0.8, particularly of 0.1 to 0.6, more particularly of 0.15 to 0.5 even more particularly of 0.2 to 0.4.

[2G] The low-substituted cellulose ether spherical microparticle according to any one of the preceding items, wherein the average particle size is 1 $\mu$m to 25 $\mu$m, more preferably 1 $\mu$m to 22 $\mu$m, still more preferably 1 $\mu$m to 20 $\mu$m, and still even more preferably 7 $\mu$m to 15 $\mu$m.

[3] A cosmetic composition comprising the low-substituted cellulose ether spherical microparticle according to the item [1] or [2] and an oiling agent.

[4] The cosmetic composition according to the item [3], further comprising water.

[5] A method of producing the low-substituted cellulose ether spherical microparticle according to the item [1] or [2], comprising:

(1) mixing an alkaline aqueous solution of low-substituted cellulose ether and a water-insoluble solvent as raw materials at a volume ratio of 1:99 to 40:60 to obtain a W/O type emulsion solution of low-substituted cellulose ether;
(2) mixing an acid aqueous solution and a water-insoluble solvent as raw materials at a volume ratio of 1:99 to 40:60 to obtain a W/O type emulsion solution of acid aqueous solution; and
(3) mixing the W/O type emulsion solution of low-substituted cellulose ether and the W/O type emulsion solution of acid aqueous solution to obtain the low-substituted cellulose ether spherical microparticle according to the item [1] or [2].

[6] The method of producing the low-substituted cellulose ether spherical microparticle according to the item [5], wherein the raw materials in the step (1) further comprise a surfactant and/or the raw materials in the step (2) further comprise a surfactant.

[7] The method of producing the low-substituted cellulose ether spherical microparticle according to the item [5], wherein the water-insoluble solvent is each independently at least a water-insoluble solvent selected from the group consisting of a silicone oil and a hydrocarbon-based solvent having a carbon number of 5 to 10.

Effects of the Invention

According to the present invention, it can be achieved to obtain a cosmetic composition with a pleasant tactile sensation by using the low-substituted cellulose ether spherical microparticle having physical properties including the average particle size of primary particles, sphericity and surface smoothness in the specified ranges. In particular, the cosmetic composition using the spherical microparticle can be excellent in application and moisture retention properties on the skin.

Description of Embodiments

While each embodiment of the present invention will now be described in detail, the present invention may take various forms to the extent that its objective can be achieved.

Unless otherwise specified, each term used herein is used in the meaning commonly used by those skilled in the art, in particular in the cosmetic field, the chemistry field and other fields, and should not be construed to have any meaning that is unduly limiting. Also, any speculations and theories herein are made on the basis of the knowledge and experiences of the present inventors and as such, the present invention is not bound by any such speculations and theories.

While the term "composition" is not particularly limited and means composition as well known, it is, for example, composed of a combination of two or more components (raw materials). Each component may be either one alone or a combination of two or more.

The term "and/or" as used herein means either any one of, any combination of two or more of, or combination of all of listed related items.

The term "content" is synonymous with concentration and amount used (amount added), and means the ratio of the amount of a component relative to the total amount of the composition. However, the total amount of the components may not exceed 100%.

The wording "to" for indicating a range of values is intended to include values preceding and following the symbol, and also includes a range included thereby without one of the limit values. For example, "0% to 100%" can be greater than or equal to 0%, less than or equal to 100%, and between 0% and 100%. The terms "more than" and "less than" used herein means the lower and upper limits without including a value following the term, respectively. For example, "more than 1" means a value beyond 1, and "less than 100" means a value below 100. The term "about" means an amount within $\pm 10\%$ of the numerical quantity following the term. For example, "about 100" means $100 \pm 10\%$, i.e., means from 90 to 110. The terms "include," "comprise," and "contain" mean that an element(s) other than an element(s) as explicitly indicated can be added as inclusions, which are, for example, synonymous with "at least include," but encompasses the meaning of "consist of" and "substantially consist of". In other words, the terms may mean, for example, to include an element(s) as explicitly indicated as well as any one element or any two or more elements, to consist of an element(s) as explicitly indicated, or substantially consist of an element(s) as explicitly indicated. Such elements include limitations such as components, steps, conditions, and parameters.

The number of digits of an integer equals to its significant figure. For example, 1 has one significant figure and 10 has two significant figures. For a decimal number, the number of digits after a decimal point equals to its significant figure. For example, 0.1 has one significant figure and 0.10 has two significant figures.

The term "skin feel" of the cosmetic composition means that there is little stickiness or other discomfort to the skin when the cosmetic composition is applied and spread on the skin. The term "spreading behavior on the skin" of the cosmetic composition refers to the ability of the cosmetic composition to be spread smoothly on the skin without applying a large force when the cosmetic composition is applied and spread on the skin.

The term "moisture retention property" of the cosmetic composition refers to having a moisturizing feeling on the skin five minutes after the cosmetic composition is applied on the skin.

[Low-substituted cellulose ether spherical microparticle]

One aspect of the present invention is a low-substituted cellulose ether spherical microparticle. One embodiment of the low-substituted cellulose ether spherical microparticle is characterized by the average particle size ($D_{50}$) of primary

particles on a volume basis by a dry laser diffraction method, the sphericity and the surface smoothness within the predetermined ranges.

Low-substituted cellulose ether is insoluble in water but soluble in an alkaline solution. In general, cellulose is insoluble in water. In contrast, cellulose ether in which the hydrogen atoms of hydroxyl groups on glucose rings constituting cellulose are substituted with an alkyl group, a hydroxyalkyl group and/or any other functional group becomes soluble in water depending on the degree of functional group substitution. However, the low-substituted cellulose ether in which the above-described substitution degree is low tends to have a property of being insoluble in water, but instead being soluble in an alkaline solution. In addition, when an acid is added to an alkaline solution of low-substituted cellulose ether and then the mixture is neutralized and coagulated, low-substituted cellulose ether can be regenerated from the alkaline solution. The resulting low-substituted cellulose ether is not soluble in water, but can have a property of absorbing water and swelling.

In summary, low-substituted cellulose ether can have the following properties (1) to (4): (1) being insoluble in water, (2) absorbing water and swelling, (3) being soluble in an alkaline solution, and (4) being regenerated from an alkaline solution by neutralization coagulation treatment using an acid.

The low-substituted cellulose ether spherical microparticle according to one embodiment of the present invention has an average particle size ($D_{50}$) of primary particles on a volume basis by a laser diffraction method of 1 $\mu$m to 30 $\mu$m, a sphericity of 0.75 to 1.0, and a surface smoothness of 75% to 100%. As used herein, the term "spherical microparticle" refers to one having the average particle size of primary particles, sphericity and surface smoothness within the above-described ranges. In other words, if any one of the average particle size of primary particles, sphericity and surface smoothness falls out of the above-described ranges, such an entity is not called the "spherical microparticle".

Examples of low-substituted cellulose ether constituting the low-substituted cellulose ether spherical microparticle include low-substituted hydroxypropyl cellulose, low-substituted hydroxyethyl cellulose, low-substituted methyl cellulose, and low-substituted hydroxypropylmethyl cellulose. From the viewpoint of favorable alkali solubility and water absorbing and swelling, the low-substituted hydroxypropyl cellulose is preferable.

The low-substituted cellulose ether spherical microparticle according to one embodiment of the present invention may have an average particle size ($D_{50}$) of primary particles on a volume basis by a laser diffraction method of 1 $\mu$m to 30 $\mu$m as an average particle size of primary particles. From the viewpoint of favorable application and moisture retention properties on the skin when used in a cosmetic composition, examples of the average particle size includes preferably 1 $\mu$m to 25 $\mu$m, more preferably 1 $\mu$m to 22 $\mu$m, still more preferably 1 $\mu$m to 20 $\mu$m, and still even more preferably 7 $\mu$m to 15 $\mu$m. The average particle size of primary particles is determined according to the method described in the section <Average particle size of primary particles> in Examples described below, in which a powder sample is jetted with compressed air and the jetted sample is irradiated with a laser beam to obtain diffraction intensity, and then the average particle size is determined based on the diffraction intensity as an average particle size on a volume basis.

The low-substituted cellulose ether spherical microparticle according to one embodiment of the present invention may have a sphericity of 0.75 to 1.00. From the viewpoint of favorable application and moisture retention properties on the skin when used in a cosmetic composition, examples of the sphericity include preferably 0.78 to 1.00, more preferably 0.80 to 1.00, still more preferably 0.82 to 1.00, and even still more preferably 0.85 to 1.00. The sphericity is determined by the method described in the section <Sphericity> in Examples described below.

The low-substituted cellulose ether spherical microparticle according to one embodiment of the present invention may have a surface smoothness of 75% to 100%. From the viewpoint of favorable application and moisture retention properties on the skin when used in a cosmetic composition, examples of the surface smoothness include preferably 78% to 100%, more preferably 80% to 100%, still more preferably 82% to 100%, and even still more preferably 85% to 100%. The surface smoothness is determined by the method described in the section <Surface smoothness> in Examples described below.

The low-substituted cellulose ether spherical microparticle according to one embodiment of the present invention may have any other physical properties in addition to the average particle size of primary particles, sphericity and surface smoothness as long as the other physical properties do not interfere with the solution to the problem of the present invention. The other physical properties are not particularly limited, but include, for example, aspect ratio, substituent content and molar substitution.

Of the low-substituted cellulose ether spherical microparticle according to one embodiment of the present invention, examples of the aspect ratio include preferably 1.00 to 1.30, more preferably 1.00 to 1.20, and still more preferably 1.00 to 1.15, in association with high sphericity. The aspect ratio is determined by the method described in the section <Aspect ratio> in Examples described below.

Of the low-substituted cellulose ether spherical microparticle according to one embodiment of the present invention, examples of the molar substitution include preferably 0.05 to 1.0, more preferably 0.05 to 0.8, and still more preferably 0.1 to 0.6, 0.1 to 0.5 or 0.1 to 0.4, from a standpoint of water insolubility and alkali solubility. The molar substitution refers to the total number of the average molar numbers of hydroxyalkoxy and alkyl groups per mole of anhydrous glucose. For example, the molar substitution of low-substituted hydroxypropyl cellulose spherical microparticle is determined by converting a value measured by the quantitative method in the section "Low substituted hydroxypropyl cellulose" described in Japanese Pharmacopoeia, 18th Edition. The molar substitution of low-substituted cellulose ether spherical microparticle corresponds to the molar substitution of the low-substituted cellulose ether that is the raw material.

[Method of producing low-substituted cellulose ether spherical microparticle]

The low-substituted cellulose ether spherical microparticle according to one embodiment of the present invention can be produced, for example, by on the one hand mixing an alkaline aqueous solution of low-substituted cellulose ether and a water-insoluble solvent at a volume ratio of 1:99 to 40:60 to obtain an water-in-oil (W/O) type emulsion solution of low-substituted cellulose ether while on the other hand mixing an acid aqueous solution and a water-insoluble solvent at a volume ratio of 1:99 to 40:60 to obtain a W/O type emulsion solution of acid aqueous solution, and then mixing these emulsion solutions.

Another aspect of the present invention is a method of producing the low-substituted cellulose ether spherical microparticle according to one embodiment of the present invention. The production method according to one embodiment of the present invention includes the following steps (1) to (3):

(1) mixing an alkaline aqueous solution of low-substituted cellulose ether and a water-insoluble solvent as raw materials at a volume ratio of 1:99 to 40:60 to obtain a W/O type emulsion solution of low-substituted cellulose ether;
(2) mixing an acid aqueous solution and a water-insoluble solvent as raw materials at a volume ratio of 1:99 to 40:60 to obtain a W/O type emulsion solution of acid aqueous solution; and
(3) mixing the W/O type emulsion solution of low-substituted cellulose ether and the W/O type emulsion solution of acid aqueous solution to obtain the low-substituted cellulose ether spherical microparticle according to one embodiment of the present invention.

In the production method according to one embodiment of the present invention, the step (1) may be carried out followed by the step (2), or the step (2) may be carried out followed by the step (1), or the steps (1) and (2) may be carried out in parallel. The step (3) is carried out after the steps (1) and (2).

[Step (1): Preparation of W/O type emulsion solution of low-substituted cellulose ether]

The main raw materials in the step (1) are an alkaline aqueous solution of low-substituted cellulose ether and a water-insoluble solvent.

The alkaline aqueous solution of low-substituted cellulose ether is obtained by dissolving low-substituted cellulose ether uniformly in an alkaline aqueous solution.

Examples of alkali in the alkaline aqueous solution include hydroxide salts such as sodium hydroxide, potassium hydroxide and ammonium hydroxide; organic amine bases such as monoethanolamine, aminomethyl propanol, aminomethyl propanediol, tromethamine and tetrahydroxypropyl ethylenediamine, preferably sodium hydroxide in view of the solubility of low-substituted cellulose ether.

Examples of the alkali concentration of the alkaline aqueous solution include preferably 0.1% by mass to 30% by mass, more preferably 0.3% by mass to 25% by mass, still more preferably 0.5% by mass to 20% by mass, and even still more preferably 1% by mass to 15% by mass from a standpoint of solubility of low-substituted cellulose ether.

Examples of the content of low-substituted cellulose ether in the alkaline aqueous solution of low-substituted cellulose ether include preferably 0.1% by mass to 20% by mass, more preferably 0.5% by mass to 17% by mass, still more preferably 1% by mass to 15% by mass, and even still more preferably 1% by mass to 10% by mass or 3% by mass to 7% by mass, in view of the viscosity of alkaline solution.

The water-insoluble solvent may be an oil-based solvent which has extremely low or no compatibility with water. Examples of the solvent include silicone oils and hydrocarbon-based solvents having a carbon number of 5 to 10.

Examples of the silicone oil include linear or branched organopolysiloxane having low to high viscosity such as dimethylpolysiloxane, tristrimethylsiloxymethylsilane, caprylyl methicone, phenyl trimethicone, tetrakistrimethylsilylsilane, methylphenylpolysiloxane, methylhexylpolysiloxane, methylhydrogenpolysiloxane and dimethylsiloxane-methylphenylsiloxane copolymer; cyclic organopolysiloxane such as octamethylcyclotetrasiloxane, decamethylcyclopentasiloxane, dodecamethylcyclohexasiloxane, tetramethyl tetrahydrogen cyclotetrasiloxane and tetramethyl tetraphenyl cyclotetrasiloxane; amino-modified organopolysiloxane; pyrrolidone-modified organopolysiloxane; pyrrolidone carboxylic acid-modified organopolysiloxane; higher alkoxy-modified silicone such as stearoxyl silicone; higher fatty acid-modified silicone, alkyl-modified silicone, long chain alkyl-modified silicone, amino acid-modified silicone, and fluorinated silicone.

Examples of the hydrocarbon-based solvent having a carbon number of 5 to 10 include pentane, isopentane, neopentane, hexane, cyclohexane, isohexane, heptane, octane, nonane and decane.

The alkaline aqueous solution of low-substituted cellulose ether and the water-insoluble solvent are mixed at such a ratio that a W/O type emulsion is formed. Examples of the mixing volume ratio ([alkaline aqueous solution of low-substituted cellulose ether]:[water-insoluble solvent]) include preferably 1:99 to 40:60, more preferably 3:97 to 38:62, still more preferably 5:95 to 36:64, and even still more preferably 10:90 to 35:65, from the viewpoint of forming a fine emulsion.

In the step (1), it is preferable to use a surfactant as any other raw material to stabilize the emulsion.

The surfactant is not particularly limited as long as it is a surfactant normally employed as a component in a cosmetic composition. Examples of the surfactant include nonionic surfactants, anionic surfactants, cationic surfactants, and amphoteric surfactants.

Examples of the nonionic surfactant include polyether-modified silicone; polyglycerin-modified silicone; acrylic silicone types; sorbitan fatty acid esters such as polyoxyethylene sorbitan monostearate, sorbitan oleate and sorbitan sesquiisostearate, and hardened castor oil derivatives.

Examples of the anionic surfactant include fatty acid salts such as sodium laurate; higher alkyl sulfate ester salts such as sodium lauryl sulfate; alkyl ether sulfate ester salts such as triethanolamine POE lauryl sulfate; N-acyl sarcosine salts; sulfosuccinate salts; and N-acylamino acid salts.

Examples of the cationic surfactant include alkyltrimethylammonium salts such as stearyltrimethylammonium chloride; benzalkonium chloride; and benzethonium chloride.

Examples of the amphoteric surfactant include betaine-type surfactants such as alkyl betaine and amide betaine.

Examples of the amount of surfactant used include as an external addition preferably 0.01 vol% to 10 vol%, more preferably 0.01 vol% to 9 vol%, still more preferably 0.01 vol% to 8 vol%, and even still more preferably 0.05 vol% to 0.5 vol%, relative to the total amount of the alkaline aqueous solution of low-substituted cellulose ether and the water-insoluble solvent used, from the viewpoint of stabilizing the emulsion.

Mixing raw materials is preferably carried out by agitation. The agitation may be manual or mechanical, but for stable emulsion formation, preferably mechanical agitation using a stirring and mixing device.

Examples of the stirring and mixing device include homo mixers (e.g., "Homomixer MARK II Model 2.5" manufactured by PRIMIX Corporation), homo dispers (e.g., "Homodisper Model 2.5" manufactured by PRIMIX Corporation), homogenizers (e.g., "Ace Homogenizer" manufactured by NIHONSEIKI KAISHA LTD.), AGI homomomixers (e.g., "Model 2M-03" manufactured by PRIMIX Corporation), multi-axis dispersion mixers (e.g., "COMBI MIX" manufactured by PRIMIX Corporation), and colloid mills (e.g., "Colloid Mill MM-2" manufactured by NIHONSEIKI KAISHA LTD.).

The agitation using a stirring and mixing device may be carried out under such a condition that the W/O type emulsion solution of low-substituted cellulose ether can be obtained. For example, when using a homo mixer, the rotor rotation speed during mixing is preferably 1,000 rpm to 10,000 rpm, more preferably 5,000 rpm to 10,000 rpm; and the mixing time is preferably 3 minutes to 60 minutes, and more preferably 10 to 30 minutes, from the viewpoint of forming a fine emulsion.

Any other raw materials may be used in the step (1) in addition to the alkaline aqueous solution of low-substituted cellulose ether, the water-insoluble solvent and the surfactant. However, preferably no environmental pollutants are used as any other raw material, and more preferably no carbon disulfide is used.

[Step (2): Preparation of W/O type emulsion solution of acid aqueous solution]

The main raw materials in the step (2) are an acid aqueous solution and a water-insoluble solvent.

Examples of acid in the acid aqueous solution include inorganic acids such as hydrochloric acid and sulfuric acid, and organic acids such as citric acid, oxalic acid and malic acid, preferably hydrochloric acid.

Examples of the acid concentration in the acid aqueous solution include preferably 1% by mass to 50% by mass, more preferably 3% by mass to 30% by mass, and still more preferably 5% by mass to 20% by mass from the viewpoint of stably coagulating and precipitating the low-substituted cellulose ether spherical microparticle after mixing the W/O type emulsion solution of low-substituted cellulose ether and the W/O type emulsion solution of acid aqueous solution.

The acid aqueous solution is preferably set such that the salt concentration falls within a predetermined range from the viewpoint of facilitating formation of the low-substituted cellulose ether spherical microparticle. For example, when hydrochloric acid is employed as the acid, sodium chloride may be preferably contained in the acid aqueous solution. In this case, the acid aqueous solution may contain sodium chloride such that the final concentration becomes preferably 1% by mass to 20% by mass, and more preferably 5% by mass to 15% by mass.

As for the water-insoluble solvent, those described in the step (1) may be referenced. The water-insoluble solvent may be the same as or different from that used in the step (1), but preferably the same as that used in the step (1) because of stably forming the low-substituted cellulose ether spherical microparticle.

The acid aqueous solution and the water-insoluble solvent are mixed at such a ratio that the W/O type emulsion is formed. Examples of the mixing volume ratio ([acid aqueous solution]:[water-insoluble solvent]) include preferably 1:99 to 40:60, more preferably 3:97 to 38:62, still more preferably 5:95 to 36:64, and even still more preferably 10:90 to 35:65 in view of forming a fine emulsion.

In the step (2), as in the step (1), a surfactant is preferably used as any other raw material to stabilize the emulsion.

As for the surfactant, those described in the step (1) may be referenced. The surfactant may be the same as or different from that used in the step (1), but preferably the same as that used in the step (1) because of stably forming the low-substituted cellulose ether spherical microparticle.

Examples of the amount of surfactant used include as an external addition preferably 0.01 vol% to 10 vol%, more preferably 0.01 vol% to 9 vol%, still more preferably 0.01 vol% to 8 vol%, and even still more preferably 0.05 vol% to 0.5 vol%, relative to the total amount of the acid aqueous solution and the water-insoluble solvent used from the viewpoint of stabilizing the emulsion.

Mixing raw materials is preferably carried out by agitation. The agitation may be manual or mechanical, but for stable emulsion formation, mechanical agitation using a stirring and mixing device is preferred. As for the types and usage conditions of the stirring and mixing device, those described in the step (1) may be referenced.

Any other raw materials may be used in the step (2) in addition to the acid aqueous solution, the water-insoluble solvent and the surfactant. However, preferably no environmental pollutants are used as any other raw material, and more preferably no carbon disulfide is used.

[Step (3): Formation of low-substituted cellulose ether spherical microparticle]

The main raw materials in the step (3) are the W/O type emulsion solution of low-substituted cellulose ether, which is the product of the step (1), and the W/O type emulsion solution of acid aqueous solution, which is the product of the step (2).

The W/O type emulsion solution of low-substituted cellulose ether and the W/O type emulsion solution of acid aqueous solution are mixed at such a ratio that the low-substituted cellulose ether spherical microparticle is formed. Examples of the mixing volume ratio ([W/O type emulsion solution of low-substituted cellulose ether]:[W/O type emulsion solution of acid aqueous solution]) include preferably 20:80 to 80:20, more preferably 30:70 to 70:30, and still more preferably 40:60 to 60:40 in view of forming a fine spherical particle.

The W/O type emulsion solution of low-substituted cellulose ether and the W/O type emulsion solution of acid aqueous solution are mixed such that the low-substituted cellulose ether spherical microparticle is formed. For example, the mixing of the W/O type emulsion solution of low-substituted cellulose ether and the W/O type emulsion solution of acid aqueous solution may be preferably carried out by gradually adding the W/O type emulsion solution of low-substituted cellulose ether to the W/O type emulsion solution of acid aqueous solution while mechanically stirring the W/O type emulsion solution of acid aqueous solution using a stirring and mixing device from the viewpoint of yielding a fine spherical particle.

As for the stirring and mixing device, those described in the step (1) may be referenced.

Agitation using a stirring and mixing device may be carried out under such a condition that the low-substituted cellulose ether spherical microparticle can be obtained. For example, when using a homo mixer, the rotor rotation speed during mixing is preferably 1,000 rpm to 10,000 rpm, and more preferably 2,000 rpm to 4,000 rpm in view of yielding a fine spherical particle.

Examples of the time of adding the W/O type emulsion solution of low-substituted cellulose ether to the W/O type emulsion solution of acid aqueous solution include preferably 3 minutes to 60 minutes, and more preferably 10 minutes to 30 minutes, in view of forming a fine spherical particle.

In the step (3), after adding the W/O type emulsion solution of low-substituted cellulose ether to the W/O type emulsion solution of acid aqueous solution, the resulting mixture may be preferably further stirred. The stirring condition for the mixture of the W/O type emulsion solution of acid aqueous solution and the W/O type emulsion solution of low-substituted cellulose ether may be such a condition that the low-substituted cellulose ether spherical microparticle can be sufficiently coagulated and precipitated. For example, when using the stirring and mixing device described above or other devices such as a magnetic stirrer, the rotation speed is preferably 500 rpm to 3,000 rpm, and the stirring time is preferably 30 minutes to 5 hours, and more preferably 2 hours to 5 hours.

The low-substituted cellulose ether spherical microparticle coagulated and precipitated may be recovered, for example, by subjecting the resulting mixture to solid-liquid separation treatment such as filtration treatment. The low-substituted cellulose ether spherical microparticle recovered may be subjected to any other processing treatments such as drying treatment, as appropriate.

[Cosmetic composition]

Another aspect of the present invention is a cosmetic composition. The cosmetic composition according to one embodiment of the present invention is characterized by containing as components the low-substituted cellulose ether spherical microparticle according to one embodiment of the present invention and an oiling agent.

The cosmetic composition according to one embodiment of the present invention contains the low-substituted cellulose ether spherical microparticle according to one embodiment of the present invention, so that the cosmetic composition can have a favorable skin feel, and spreading behavior and moisture retention property on the skin, and comprehensively an excellent tactile sensation.

The content of the low-substituted cellulose ether spherical microparticle is not particularly limited. Examples of the content include preferably 0.01% by mass to 20% by mass, more preferably 0.1% by mass to 15% by mass, still more preferably 0.5% by mass to 10% by mass, and even still more preferably 1% by mass to 7% by mass, relative to the total amount of the cosmetic composition, from the viewpoint of application property on the skin of the cosmetic composition.

The oiling agent may be any oiling agent generally used in producing a cosmetic composition. Examples of the oiling agent include hydrocarbon oils, ester oils, animal and vegetable oils and fats, and silicone oils.

Examples of the hydrocarbon oil include fluid paraffin, stearic acid, hydrogenated polyisobutene, hydrogenated polydecene, squalane, squalene, pristane, light isoparaffin, light fluid isoparaffin, heavy fluid isoparaffin, fluid isoparaffin, tetradecene, isohexadecane, isododecane and alpha olefin oligomers.

Examples of the ester oil include glyceryl stearate, ethyl oleate, ethyl linoleate, isopropyl myristate, isopropyl palmitate, isopropyl isostearate, cetyl 2-ethylhexanoate, isocetyl 2-ethylhexanoate, stearyl 2-ethylhexanoate, isostearyl 2-ethylhexanoate, cetyl palmitate, 2-ethylhexyl palmitate, 2-hexyldecyl isostearate, isostearyl isostearate, trimethylolpropane triisostearate, myristyl myristate, cetyl myristate, octyldodecyl myristate, isostearyl myristate, isocetyl myristate, hexyl laurate, decyl oleate, octyldodecyl oleate, isostearyl pivalate, isopropyl isostearate, isononyl isononanoate, 2-ethylhexyl isononanoate, isodecyl isononanoate, isotridecyl isononanoate, octyldodecyl erucate, neopentyl glycol didecanoate, pentaerythrityl tetraethylhexanoate, diisostearyl malate, trimethylolpropane triethylhexanoate, didecyl adipate, cholesteryl isostearate, batyl isostearate, hardened castor oil monohydroxy stearate, lanolin fatty acid isostearyl, lanolin fatty acid isopropyl, lanolin fatty acid octyldodecyl, cetyl ricinoleate, dioctyl succinate, cetyl lactate, propylene glycol dicaprylate, propylene glycol dicaprate, propylene glycol dinonane, di(caprylic/capric) acid propylene glycol, propylene glycol diisostearate, propylene glycol dioleate, triglycerides, animal and vegetable oils. Examples of triglyceride include triglycerides comprised of glycerin and fatty acids such as caproic acid, caprylic acid, capric acid, 2-ethylhexanoic acid, isotridecanoic acid, isopalmitic acid, isostearic acid, eicosanoic acid and oleic acid.

Examples of the animal and vegetable oil and fat include liquid lanolin, olive oil, sunflower oil, safflower oil, castor oil and camellia oil.

Examples of the silicone oil include linear or branched organopolysiloxane having low to high viscosity, such as dimethylpolysiloxane, tristrimethylsiloxymethylsilane, caprylyl methicone, phenyl trimethicone, tetrakistrimethylsiloxysilane, methylphenylpolysiloxane, methylhexylpolysiloxane, methylhydrogenpolysiloxane, dimethylsiloxane-methylphenylsiloxane copolymer; cyclic organopolysiloxane such as octamethylcyclotetrasiloxane, decamethylcyclopentasiloxane, dodecamethylcyclohexasiloxane, tetramethyltetrahydrogencyclotetrasiloxane, tetramethyltetraphenylcyclotetrasiloxane; amino modified organopolysiloxane; pyrrolidone modified organopolysiloxane; pyrrolidone carboxylic acid-modified organopolysiloxane; higher alkoxy-modified silicone such as stearoxyl silicone; higher fatty acid-modified silicone, alkyl-modified silicone, long-chain alkyl-modified silicone, amino acid-modified silicone, and fluorinated silicone.

The content of the oiling agent is not particularly limited. Examples of the content include preferably 0.1% by mass to 50% by mass, more preferably 0.3% by mass to 45% by mass, still more preferably 0.5% by mass to 40% by mass, and even still more preferably 1% by mass to 35% by mass, relative to the total amount of the cosmetic composition, from the viewpoint of moisture retention property of the cosmetic composition.

The cosmetic composition of the present invention may further contain water.

Water is not particularly limited as long as water is one normally used in the process of manufacturing a cosmetic composition. Examples of water include tap water and purified water, preferably purified water because of not containing impurities.

The content of water is not particularly limited as long as the content is an amount normally used in a cosmetic composition depending on the dosage form of the cosmetic composition. Examples of the content include preferably 90% by mass or less, more preferably 85% by mass or less, still more preferably 80% by mass or less, and even still more preferably 75% by mass or less, relative to the total amount of the cosmetic composition, form the viewpoint of application property on the skin of the cosmetic composition. The lower limit of the content may be typically 0% by mass. Taken together, the content of water is preferably 0% by mass to 90% by mass, more preferably 10% by mass to 85% by mass, still more preferably 20% by mass to 80% by mass, and even still more preferably 30% by mass to 75% by mass.

The cosmetic composition may further contain any other components to have desired properties in addition to the low-substituted cellulose ether spherical microparticle, the oiling agent and water. Examples of the other component include additives such as solvents, surfactants, pH adjusting agents, clay minerals, colorants, thickening agents, antioxidants, preservative agents, moisturizers, pearlizing agents, astringents, whitening agents, and fragrances. Examples of the additives are listed below.

Examples of the solvent include polyhydric alcohols such as glycerin, diglycerin, butylene glycol, propylene glycol and dipropylene glycol, and alcohols such as ethanol.

The surfactant is not particularly limited as long as the surfactant is one normally used as a component in a cosmetic composition. As for the surfactant, those explained in the step (1) may be referenced.

Examples of the pH adjusting agent include ethanolamine, diethanolamine, triethanolamine, citric acid, sodium citrate, gluconic acid, succinic acid, sodium hydroxide and potassium hydroxide.

Examples of the clay mineral include talc, mica, sericite, kaolin, montmorillonite, saponite, hectorite and smectite.

Examples of the colorant include red iron oxide, yellow iron oxide, black iron oxide, titanium oxide, ultramarine blue, iron blue, manganese violet, cobalt violet, chromium hydroxide, chromium oxide, cobalt oxide, cobalt titanate, iron oxide doped titanium oxide, iron titanate, (titanium/titanium oxide) calcined products, (Li/cobalt) titanate, cobalt titanate, titanium nitride, iron hydroxide, inorganic brown pigments such as γ-iron oxide, inorganic yellow pigments such as ochre, and colored pigments such as tar pigments raked and natural pigments raked. The pigment may be in any shape such as spherical shape, approximately spherical shape, rod, spindle, petal, strip and irregular shape. The geometry of the pigment is no particularly limited as long as the pigment can color the formulation.

Examples of the thickening agent include water-soluble polymers such as xanthane gum, guar gum, gellan gum, locust bean gum, carboxymethyl cellulose, hydroxyethyl cellulose, hydroxypropylmethyl cellulose, hydrophobized hydroxypropylmethyl cellulose, methylcellulose, cationized hydroxyethyl cellulose, carboxyvinyl polymer and polyvinyl alcohol.

Examples of the antioxidant include tocopherol, tocopherol acetate, butyl hydroxyanisole and dibutyl hydroxytoluene.

Examples of the preservative agent include methylparaben, ethylparaben, propylparaben, butylparaben and phenoxyethanol.

Examples of the moisturizer include propylene glycol, hyaluronic acid, sodium hyaluronate, polyethylene glycol, mucopolysaccharide, urea, sorbitol, chondroitin sulfate, pyrrolidone carboxylic acid, sodium lactate and polyaspartic acid.

Examples of the pearlizing agent include ethylene glycol monostearate, ethylene glycol monobehenate, ethylene glycol distearate and ethylene glycol dibehenate.

Examples of the astringent include zinc oxide, zinc paraphenol sulfonate, aluminum hydroxychloride, allantoin dihydroxyaluminum, peppermint extract, aloe extract, hamamelis extract, rosemary extract, lavender extract and eucalyptus extract.

Examples of the whitening agent include arbutin, α-arbutin, ascorbic acid; ascorbic acid fatty acid esters such as sodium ascorbate phosphate, magnesium ascorbate phosphate and tetraisopalmitate ascorbate; kojic acid, ellagic acid and tranexamic acid; and the derivatives thereof.

Examples of the fragrance include natural fragrances and synthetic fragrances.

Examples of the natural fragrance include rose oil, jasmine oil, lavender oil, ylang-ylang oil, peppermint oil, geranium oil, patchouli oil, sandalwood oil, cinnamon oil, lemon oil, orange oil and bergamot oil.

Examples of the synthetic fragrance include limonene, β-caryophyllene, cis-3-hexenol, linalool, farnesol, β-phenylethyl alcohol, 2,6-nonadienal, citral, α-hexyl cinnamaldehyde, iota-carvone, cyclopentadecanone, linalyl acetate, γ-undecalactone, aurantiol and I-menthol.

While the content of the additives is not particularly limited, examples of the content include preferably 0.1% by mass to 95% by mass, more preferably 0.5% by mass to 50% by mass, and still more preferably 10% by mass to 40% by mass, from the viewpoint of skin feel, spreading behavior and moisture retention property on the skin imparted to the cosmetic composition.

The method of producing the cosmetic composition is not particularly limited. The cosmetic composition may be obtained, for example, by adding a water-soluble component to water and mixing them to obtain an aqueous solution, then adding a solid component containing the low-substituted cellulose ether spherical microparticle to the resulting aqueous solution and mixing them to obtain a mixture, then adding an oiling agent to the resulting mixture, and mixing them, and then subjecting the resulting mixture to any additional processing treatments such as drying treatment, as appropriate.

The use mode and dosage form of the cosmetic composition according to one embodiment of the present invention is not particularly limited. Examples of the use mode and dosage form include makeup cosmetics, skin care cosmetics, fragrance cosmetics, body care cosmetics, and other cosmetics, in particular creams, skin milks, foundations, skin lotions, serums, sunscreen milks, packs, face washing creams, hand creams, makeup cleansers, makeup bases, concealers, cheek rouges, eye shadows, eyeliners, eyebrows, lipsticks, sunscreen creams, hair removal creams, all-in-one cosmetics. The cosmetic composition according to one embodiment of the present invention may be impregnated into sheets and sprayed when used. Therefore, the cosmetic composition according to one embodiment of the present invention may be expected to be in the dosage forms such as impregnated sheets, impregnated masks and sprays such as pumps.

Examples

While the present invention will now be described in further detail with reference to Examples and Comparative Examples, the present invention is not limited to Examples as below. Unless otherwise noted, each operation was performed at 25°C.

Table 1 shows the low-substituted cellulose ethers (CE) used in Examples and Comparative Examples, which are granular low-substituted hydroxypropyl cellulose.

[Table 1]

| Type of CE | Molar substitution | Average particle size (μm) | Aspect ratio (-) |
|---|---|---|---|
| CE-1 | 0.26 | 55 | 2.5 |
| CE-2 | 0.26 | 45 | 3.8 |
| CE-3 | 0.18 | 45 | 3.8 |
| CE-4 | 0.26 | 55 | 5 |
| CE-5 | 0.26 | 20 | 3.6 |

The molar substitution of the low-substituted cellulose ether was determined by converting a value measured by the quantitative method in the section "Low substituted hydroxypropyl cellulose" described in Japanese Pharmacopoeia, 18th Edition.

The average particle size and aspect ratio of the low-substituted cellulose ether were determined by the methods described in the sections <Average particle size of primary particles> and <Aspect ratio> as described below, respectively.

[Example 1]

<Preparation of alkaline aqueous solution of low-substituted cellulose ether>

To a 500 ml-volume beaker, 190 g of 5% by mass sodium hydroxide aqueous solution was added, and the beaker was cooled in a water bath until reaching a temperature of 5 °C. Then, while stirring the 5% by mass sodium hydroxide aqueous solution, 10 g of CE-1 as the low-substituted cellulose ether was added thereto, and the stirring was carried out until CE-1 was uniformly dissolved to prepare an alkaline aqueous solution of low-substituted cellulose ether in which the content of CE-1 was 5% by mass.

<Preparation of W/O type emulsion solution of low-substituted cellulose ether>

To a 500 ml-volume beaker, 90 ml of silicone oil KF-96L-1.5cs (Shin-Etsu Chemical), 10 ml of the alkaline aqueous solution of low-substituted cellulose ether with CE-1 of 5% by mass, and 0.1 ml of polyether modified silicone KF-6017 (Shin-Etsu Chemical) as a surfactant were added. These raw materials were then stirred using the homo-mixer ("Homomixer MARK II Model 2.5" manufactured by PRIMIX Corporation; rotor diameter 30.0 mm) at a rotation speed of 8,000 rpm for 15 minutes to prepare a W/O type emulsion solution of low-substituted cellulose ether.

<W/O type emulsion solution of acid aqueous solution>

To a 500 ml-volume beaker, 90 ml of silicone oil KF-96L-1.5cs (Shin-Etsu Chemical), an aqueous solution in which sodium chloride was dissolved at the final concentration of 10% by mass in 10 ml of 10% by mass hydrochloric acid aqueous solution, and 0.1 ml of polyether modified silicone KF-6017 (Shin-Etsu Chemical) as a surfactant were added. These raw materials were then stirred using the homo-mixer ("Homomixer MARK II Model 2.5" manufactured by PRIMIX Corporation; rotor diameter 30.0 mm) at a rotation speed of 8,000 rpm for 15 minutes to prepare a W/O type emulsion solution of acid aqueous solution.

<Formation of low-substituted cellulose ether spherical microparticle>

While stirring 100 ml of the W/O type emulsion solution of acid aqueous solution at 3,000 rpm using the homo mixer ("Homomixer MARK II Model 2.5" manufactured by PRIMIX Corporation; rotor diameter 30.0 mm), 100 ml of the W/O type emulsion solution of low-substituted cellulose ether was added thereto over 15 minutes. The resulting mixture was then stirred at 2,000 rpm for 3 hours using a magnetic stirrer to precipitate a low-substituted cellulose ether spherical microparticle.

The resulting suspension containing the low-substituted cellulose ether spherical microparticle was placed in a 300 ml-volume separatory funnel, and after leaving to stand, the lower aqueous layer containing the low-substituted cellulose ether spherical microparticle was collected. The obtained aqueous layer was filtered through Kiriyama funnel set with No. 5A filter paper, and the residue was washed with water and ethanol, and then subjected to air-drying for evaporation of ethanol to obtain a low-substituted cellulose ether spherical microparticle (MB-1).

[Examples 2 to 5]

The low-substituted cellulose ether spherical microparticles MB-2 to 5 were prepared in the same manner as in Example 1, except that the low-substituted cellulose ether used was changed from CE-1 to CE-2 to CE-5, respectively.

[Example 6]

The low-substituted cellulose ether spherical microparticle MB-6 was prepared in the same manner as in Example 1, except that in the preparation of the W/O type emulsion solution of low-substituted cellulose ether, the amount of the alkaline aqueous solution of low-substituted cellulose ether was changed to 20 ml, and the amount of silicone oil KF-96L-1.5cs was changed to 80 ml, and in the preparation of the W/O type emulsion solution of acid aqueous solution, the amount of the 10% by mass hydrochloric acid aqueous solution (with sodium chloride dissolved at a concentration of 10% by mass) was changed to 20 ml, and the amount of silicone oil KF-96L-1.5cs was changed to 80 ml.

[Example 7]

The low-substituted cellulose ether spherical microparticle MB-7 was prepared in the same manner as in Example 1, except that in the preparation of the W/O type emulsion solution of low-substituted cellulose ether, the amount of the alkaline aqueous solution of low-substituted cellulose ether was changed to 35 ml, and the amount of silicone oil KF-96L-1.5cs was changed to 65 ml, and in the preparation of the W/O type emulsion solution of acid aqueous solution, the amount of the 10% by mass hydrochloric acid aqueous solution (with sodium chloride dissolved at a concentration of 10% by mass) was changed to 35 ml, and the amount of silicone oil KF-96L-1.5cs was changed to 65 ml.

[Comparative Example 1]

The low-substituted cellulose ether spherical microparticle MB-8 was prepared in the same manner as in Example 1, except that in the preparation of the W/O type emulsion solution of low-substituted cellulose ether, the amount of the alkaline aqueous solution of low-substituted cellulose ether was changed to 45 ml, and the amount of silicone oil KF-96L-1.5cs was changed to 55 ml, and in the preparation of the W/O type emulsion solution of acid aqueous solution, the amount of the 10% by mass hydrochloric acid aqueous solution (with sodium chloride dissolved at a concentration of 10% by mass) was changed to 45 ml, and the amount of silicone oil KF-96L-1.5cs was changed to 55 ml.

[Comparative Example 2]

In 425 g of a 6.3% by mass sodium hydroxide aqueous solution prepared, 7.5 g of CE-1 was dissolved. The resulting solution was neutralized by dropping 40.2 g of acetic acid through a small hole in the vessel over 5 minutes while shearing and grinding at 5,000 rpm using the Ace Homogenizer (manufactured by NIHONSEIKI KAISHA LTD.). After neutralization, the solution was further subjected to shearing and grinding treatment at 10,000 rpm for 10 minutes. The resulting gel was subjected to centrifugation at 10,000 rpm for 10 minutes at 25 °C using the cooling centrifuge separator ("himac CR22N" manufactured by Eppendorf Himac Technologies). After centrifugation, the precipitate obtained by discarding the supernatant was redispersed in pure water such that the solid concentration reached 2% by mass. The resulting dispersion was subjected to spray-drying treatment by the rotary atomizer method using the spray-drying apparatus ("Mobile Minor Closed Cycle" manufactured by GEA Niro) to prepare a low-substituted cellulose ether spherical microparticle MB-9. The operating conditions were as follows: atomizer rotation speed of 28,000 rpm, drying room inlet temperature of 120°C, drying room outlet temperature of 60°C, and air supply rate of 100 kg/h.

The compositions of the W/O type emulsion solution of low-substituted cellulose ether and the W/O type emulsion solution of acid aqueous solution prepared in Examples and Comparative Examples are shown in Table 2.

[Table 2]

| | | W/O type emulsion solution of low-substituted cellulose ether | | | W/O type emulsion solution of acid aqueous solution | | |
|---|---|---|---|---|---|---|---|
| | Type of CE | Alkaline aqueous solution of low-substituted cellulose ether | Silicone oil KF-96-L-1.5cs | Surfactant KF-6017 | 10% by mass hydrochloric acid (Sodium chloride dissolved at 10% by mass) | Silicone oil KF-96-L-1.5cs | Surfactant KF-6017 |
| | (-) | (ml) | (ml) | (ml) | (ml) | (ml) | (ml) |
| Example 1 | CE-1 | 10 | 90 | 0.1 | 10 | 90 | 0.1 |
| Example 2 | CE-2 | 10 | 90 | 0.1 | 10 | 90 | 0.1 |
| Example 3 | CE-3 | 10 | 90 | 0.1 | 10 | 90 | 0.1 |
| Example 4 | CE-4 | 10 | 90 | 0.1 | 10 | 90 | 0.1 |
| Example 5 | CE-5 | 10 | 90 | 0.1 | 10 | 90 | 0.1 |
| Example 6 | CE-1 | 20 | 80 | 0.1 | 20 | 80 | 0.1 |
| Example 7 | CE-1 | 35 | 65 | 0.1 | 35 | 65 | 0.1 |
| Comparative Example 1 | CE-1 | 45 | 55 | 0.1 | 45 | 55 | 0.1 |

[Measurement of physical properties of low-substituted cellulose ether spherical microparticle]

For the resulting low-substituted cellulose ether spherical microparticles, and the spherical microparticle using a commercial cellulose as a raw material MB-10, the average particle size of primary particles, sphericity, surface smoothness, and aspect ratio were determined, respectively. The results are shown in Table 3.

<Average particle size of primary particles>

The average particle size of primary particles represented a volume-based average particle size ($D_{50}$) of primary particles measured by a dry laser diffraction method. Specifically, the average particle size was determined by measuring a particle size corresponding to 50% cumulative value of volume-based cumulative particle size distribution curve under the condition of a dispersion pressure of 1.5 bar and a scattering intensity of 2% to 10% in accordance with the dry method based on Fraunhofer diffraction theory using the laser diffraction particle size distribution analyzer ("Mastersizer 3000" manufactured by Malvern).

<Sphericity>

The spherical microparticle was imaged and observed with a scanning electron microscope (2,000x). The sphericity value of the spherical microparticle was calculated by dividing the circle equivalent perimeter (perimeter of circle having the same projected area as the particle image) by the perimeter (perimeter of the particle projected image). The number of particles measured at one time was 30 or more, and this was repeated at least 10 times to obtain the average value from the sphericity values obtained using 300 or more spherical microparticles in total.

<Surface smoothness>

The spherical microparticle was imaged and observed with a scanning electron microscope (2,000x). The surface smoothness value of the spherical microparticle was calculated in accordance with the following equation.

$$\text{Surface smoothness} = (1-(S1)/(S2)) \times 100$$

In the above equation, S2 represents an area covered by spherical microparticles in the image (projected area); and S1 represents, when a spherical microparticle in the image is superimposed on a circle having the same projected area as S2, the sum of "the area outside the outline of the circle having the same projected area as S2 and inside the outline of the spherical microparticle in the image" and "the area inside the outline of the circle having the same projected area as S2 and outside the outline of the spherical microparticle in the image".

The method for superimposing the spherical microparticle in the image on the circle having the same projected area as S2 is as follows. The spherical microparticle in the image was superimposed on the circle having the same projected area as S2 such that the overlapped area between the two images (the area inside the outline of the circle having the same projected area as S2 and inside the outline of the spherical microparticle in the image) was maximized.

The number of particles measured at one time was 30 or more, and this was repeated at least 10 times to obtain the average value from the surface smoothness values obtained using 300 or more spherical microparticles in total.

<Aspect ratio>

The aspect ratio of the low-substituted cellulose ether spherical microparticle was determined by photographing at measurable magnification randomly selected 50 microparticles using the scanning electron microscope ("JSM-6010 LA", manufactured by JEOL) and measuring the long diameter (L) and short diameter (D) of each microparticle. The aspect ratio (L/D) value was calculated from the obtained diameters, and the average value (average aspect ratio) was obtained from the calculated aspect ratio values (n = 50).

[Table 3]

| | Type of spherical particle | Raw material | Production manner | Average particle size of primary particles (μm) | Sphericity (-) | Surface smoothness (%) | Aspect ratio (-) |
|---|---|---|---|---|---|---|---|
| Example 1 | MB-1 | Low-substituted hydroxypropyl cellulose | Emulsion coagulation and precipitation | 11.5 | 0.98 | 97 | 1.06 |
| Example 2 | M B-2 | Low-substituted hydroxypropyl cellulose | Emulsion coagulation and precipitation | 10.8 | 0.98 | 93 | 10.8 |
| Example 3 | MB-3 | Low-substituted hydroxypropyl cellulose | Emulsion coagulation and precipitation | 9.9 | 0.98 | 92 | 10.8 |
| Example 4 | M B-4 | Low-substituted hydroxypropyl cellulose | Emulsion coagulation and precipitation | 14.4 | 0.95 | 91 | 1.10 |
| Example 5 | M B-5 | Low-substituted hydroxypropyl cellulose | Emulsion coagulation and precipitation | 8.1 | 0.98 | 98 | 1.05 |
| Example 6 | M B-6 | Low-substituted hydroxypropyl cellulose | Emulsion coagulation and precipitation | 13.3 | 0.97 | 96 | 1.11 |
| Example 7 | M B-7 | Low-substituted hydroxypropyl cellulose | Emulsion coagulation and precipitation | 18.9 | 0.90 | 89 | 1.14 |

(continued)

| | Type of spherical particle | Raw material | Production manner | Average particle size of primary particles (μm) | Sphericity (-) | Surface smoothness (%) | Aspect ratio (-) |
|---|---|---|---|---|---|---|---|
| Comparative Example 1 | MB-8 | Low-substituted hydroxypropyl cellulose | Emulsion coagulation and precipitation | 45.0 | 0.97 | 96 | 1.17 |
| Comparative Example 2 | MB-9 | Low-substituted hydroxypropyl cellulose | Spray-drying | 13.1 | 0.67 | 73 | 1.18 |
| | MB-10 | cellulose | | 10.6 | 0.95 | 95 | 1.10 |

As shown in Table 3, MB-1 to MB-7 of Examples 1 to 7 were the low-substituted cellulose ether spherical microparticle having an average particle size ($D_{50}$) of primary particles on a volume basis by a dry laser diffraction method of 1 μm to 30 μm, a sphericity of 0.75 to 1.0, and a surface smoothness of 75% to 100%. When the production manner employs emulsion coagulation and precipitation, fine water droplets are generated in the oil components by applying mechanical shear to the emulsion. MB-8 of Comparative Example 1 was prepared under the condition that the water content was larger when compared to the oil content, and it is assumed that due to such a condition, the average particle size of primary particles became larger since the generated fine droplets merged with each other to form large droplets, as well as no disintegration could occur and the fine droplets could not be generated. When the production manner employs spray-drying, it was found that only a product with lower sphericity and surface smoothness can be obtained, as with MB-9 of Comparative Example 2.

[Examples 8 to 16, Comparative Examples 3 to 4, and Reference Example 1]

<Preparation of cosmetic composition>

Using the spherical microparticles of MB-1 to MB-10, the cosmetic compositions of Examples 8 to 16, Comparative Examples 3 to 4, and Reference Example 1, which were a mock liquid foundation, were prepared according to the formulations as shown in Table 4 by the method described below. Here, the spherical microparticle of MB-10 employed cellulose as a raw material.

Propylene glycol, polyoxyethylene sorbitan monostearate, and triethanolamine were added to purified water, and the mixture was subjected to agitation treatment at 5,000 rpm for 5 minutes using the Ace Homogenizer (manufactured by NIHONSEIKI KAISHA LTD.). To the resulting treated solution, the low-substituted cellulose ether spherical microparticle, talc, titanium dioxide, red iron oxide, yellow iron oxide, and black iron oxide were added, and the mixture was subjected to agitation treatment at 70 °C for 5 minutes at 5,000 rpm using the Ace homogenizer. To the resulting treated solution, the oiling agent obtained by heating and dissolving stearic acid, glyceryl stearate, liquid lanolin, and liquid paraffin at 70 °C was added. The resulting mixture was subjected to agitation treatment at 70 °C for 5 minutes at 5,000 rpm using the Ace homogenizer to obtain a liquid cosmetic composition.

[Table 4]

| Component name | | Product name | Manufacturer | % by mass |
|---|---|---|---|---|
| Spherical particles (MB-1 to 10) | | - | - | The amounts shown in Table 5 |
| Clay mineral | Talc | Talc JA-46R | ASADA MILLING | 5 |

(continued)

| Component name | | Product name | Manufacturer | % by mass |
|---|---|---|---|---|
| Colorant | Titanium oxide | STR-100N | SAKAI CHEMICAL INDUSTRY | 2 |
| | Red iron oxide | R-516HP | Titan Kogyo | 1 |
| | Yellow iron oxide | LL-100HP | Titan Kogyo | 0.1 |
| | Black iron oxide | BL-100HP | Titan Kogyo | 0.01 |
| Surfactant | Polyoxyethylene sorbitan monostearate | Nonion ST-206 | NOF CORPORATION | 0.01 |
| pH adjusting agent | Triethanolamine | Ethanolamine Care | BASF Japan | 1 |
| Moisturizer | Propylene glycol | Reagent | Fujifilm Wako Pure Chemical | 2 |
| Oiling agent | Stearic acid | 63 Stearin S | Kawaken Fine Chemicals | 2 |
| | Glyceryl stearate | Cucina GMSV | BASF Japan | 3 |
| | Liquid lanolin | Liquid Lanolin | Nippon Fine Chemicals | 5 |
| | Liquid paraffin | Moresco White P-55 | MORESCO | 5 |
| Purified water | | - | - | Residual |
| Total | | | | 100 |

<Sensory evaluation of cosmetic composition>

The resulting cosmetic composition was subjected to sensory evaluation by five panelists who have excelled in evaluating tactile sensation of cosmetics, in terms of skin feel, and spreading behavior and moisture retention property on the skin. According to the following evaluation criteria, the cosmetic composition was scored out of five points per each panelist, and the average score was calculated from the total score of the five panelists. Finally, the cosmetic composition in which all the average scores in terms of skin feel, and spreading behavior and moisture retention property on the skin are 3.0 or more was rated as "+" while the cosmetic composition in which any one of the average scores was less than 3.0 was rated as "-". The results are shown in Table 5.

5: Very good

4: Good

3: Normal

2: Bad

1: Very bad

[Table 5]

| | Spherical microparticle | | Sensory evaluation | | | |
|---|---|---|---|---|---|---|
| | Type | Content (% by mass) | Skin feel | Spreading behavior | Moisture retention property | Evaluation result |
| Example 8 | MB-1 | 5 | 4.6 | 4.8 | 4.8 | + |

(continued)

| | Spherical microparticle | | Sensory evaluation | | | |
| --- | --- | --- | --- | --- | --- | --- |
| | Type | Content (% by mass) | Skin feel | Spreading behavior | Moisture retention property | Evaluation result |
| Example 9 | MB-2 | 5 | 4.8 | 4.4 | 4.6 | + |
| Example 10 | MB-3 | 5 | 4.6 | 4.8 | 4.4 | + |
| Example 11 | MB-4 | 5 | 4.2 | 4.4 | 4.2 | + |
| Example 12 | MB-5 | 5 | 4.8 | 4.8 | 4.4 | + |
| Example 13 | MB-6 | 5 | 4.4 | 4.4 | 4.6 | + |
| Example 14 | MB-7 | 5 | 3.8 | 4 | 3.8 | + |
| Example 15 | MB-1 | 4.5 | 4.2 | 4.4 | 3.8 | + |
| Example 16 | MB-1 | 4 | 3.8 | 3.8 | 3.6 | + |
| Comparative Example 3 | MB-8 | 5 | 1.8 | 1.6 | 2.8 | - |
| Comparative Example 4 | MB-9 | 5 | 2.8 | 2.6 | 3.2 | - |
| Reference Example 1 | MB-10 | 5 | 3.2 | 3.4 | 2.8 | - |

As shown in Table 5, the cosmetic compositions prepared using the low-substituted cellulose ether spherical microparticle having the average particle size of primary particles, sphericity, and surface smoothness within the specified range were favorable in terms of all of the skin feel, and the spreading behavior and moisture retention property on the skin, resulting in pleasant tactile sensation.

In contrast, the cosmetic composition of Comparative Example 3 prepared using the low-substituted cellulose ether spherical microparticle with a larger average particle size of primary particles, and the cosmetic composition of Comparative Example 4 prepared using the low-substituted cellulose ether spherical microparticle with lower sphericity and surface smoothness had poor skin feel, spreading behavior or moisture retention property on the skin, resulting in unpleasant tactile sensation.

Furthermore, the cosmetic composition of Reference Example 1, which was prepared using the cellulose spherical microparticle, was inferior in tactile sensation. This would be based on the presumption that the cellulose spherical microparticle had less water absorbing and swelling abilities as compared to the low-substituted cellulose ether spherical microparticle.

Industrial applicability

The low-substituted cellulose ether spherical microparticle according to one embodiment of the present invention can be used as a component of a cosmetic composition to impart a favorable tactile sensation to the cosmetic composition. The cosmetic composition according to one embodiment of the present invention is applicable for makeup cosmetics, skin care cosmetics and the like.

Cross-reference of related applications

The present application claims the benefit of priorities to Japanese Patent Application No. 2024-078097 filed on May 13, 2024, the disclosure of which is incorporated herein by reference in its entirety. The disclosure of all the documents described herein including Patent Documents 1 to 3 is incorporated herein by reference in its entirety.

**Claims**

1. A low-substituted cellulose ether spherical microparticle, having an average particle size ($D_{50}$) of primary particles, determined on a volume basis by a dry laser diffraction method, of 1 $\mu$m to 30 $\mu$m, a sphericity of 0.75 to 1.0, and a surface smoothness of 75% to 100%.

2. The low-substituted cellulose ether spherical microparticle according to claim 1, further having a molar substitution of low-substituted cellulose ether of 0.05 to 1.0.

3.  A cosmetic composition comprising the low-substituted cellulose ether spherical microparticle according to claim 1 or 2 and an oiling agent.

4.  The cosmetic composition according to claim 3, further comprising water.

5.  A method of producing the low-substituted cellulose ether spherical microparticle according to claim 1 or 2, comprising:

    (1) mixing an alkaline aqueous solution of low-substituted cellulose ether and a water-insoluble solvent as raw materials at a volume ratio of 1:99 to 40:60 to obtain a W/O type emulsion solution of low-substituted cellulose ether;
    (2) mixing an acid aqueous solution and a water-insoluble solvent as raw materials at a volume ratio of 1:99 to 40:60 to obtain a W/O type emulsion solution of acid aqueous solution; and
    (3) mixing the W/O type emulsion solution of low-substituted cellulose ether and the W/O type emulsion solution of acid aqueous solution to obtain the low-substituted cellulose ether spherical microparticle according to claim 1 or 2.

6.  The method of producing the low-substituted cellulose ether spherical microparticle according to claim 5, wherein the raw materials in the step (1) further comprise a surfactant and/or the raw materials in the step (2) further comprise a surfactant.

7.  The method of producing the low-substituted cellulose ether spherical microparticle according to claim 5, wherein the water-insoluble solvent is each independently at least a water-insoluble solvent selected from the group consisting of a silicone oil and a hydrocarbon-based solvent having a carbon number of 5 to 10.

EP 4 650 387 A1

## EUROPEAN SEARCH REPORT

**Application Number**

EP 25 17 5535

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 4 116 357 A1 (DAINICHISEIKA COLOR CHEM [JP]) 11 January 2023 (2023-01-11) | 1,3,4 | INV. C08J3/16 |
| A | * paragraphs [0031], [0053] - [0065], [0084]; claims 1-18; examples 1-8; table 1 * | 2,5-7 | A61Q19/00 C08L1/28 |
| A,D | JP 2003 252902 A (SHINETSU CHEMICAL CO) 10 September 2003 (2003-09-10) * paragraphs [0018] - [0020]; claims 1-4; examples 1-5 * | 1-7 | |
| A | JP 2022 020064 A (DAINICHISEIKA COLOR CHEM) 31 January 2022 (2022-01-31) * claims 1-3 * | 1-7 | |
| A | EP 4 357 394 A1 (DAICEL CORP [JP]) 24 April 2024 (2024-04-24) * paragraphs [0049] - [0053], [0084], [0086] - [0088], [0094]; claims 1-14; examples 1-28; tables 1-4 * | 1-7 | |
| A | JP 2006 151992 A (SHINETSU CHEMICAL CO) 15 June 2006 (2006-06-15) * paragraphs [0024] - [0033]; claims 1, 2; examples 1-8 * | 1-7 | |

**TECHNICAL FIELDS SEARCHED (IPC)**

C08J
C09J
A61Q
A61K
C08L

The present search report has been drawn up for all claims

| Place of search | Date of completion of the search | Examiner |
|---|---|---|
| Munich | 5 September 2025 | Fischer, Viktor |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

19

## ANNEX TO THE EUROPEAN SEARCH REPORT
## ON EUROPEAN PATENT APPLICATION NO.

EP 25 17 5535

05-09-2025

| Patent document cited in search report | | | Publication date | Patent family member(s) | | | Publication date |
|---|---|---|---|---|---|---|---|
| EP 4116357 | A1 | | 11-01-2023 | BR | 112022017335 | A2 | 11-10-2022 |
| | | | | CN | 115210298 | A | 18-10-2022 |
| | | | | EP | 4116357 | A1 | 11-01-2023 |
| | | | | JP | 6779400 | B1 | 04-11-2020 |
| | | | | JP | 2021138641 | A | 16-09-2021 |
| | | | | KR | 20220140900 | A | 18-10-2022 |
| | | | | US | 2023136180 | A1 | 04-05-2023 |
| | | | | WO | 2021177141 | A1 | 10-09-2021 |
| JP 2003252902 | A | | 10-09-2003 | EP | 1342733 | A1 | 10-09-2003 |
| | | | | JP | 4082656 | B2 | 30-04-2008 |
| | | | | JP | 2003252902 | A | 10-09-2003 |
| | | | | US | 2003166918 | A1 | 04-09-2003 |
| JP 2022020064 | A | | 31-01-2022 | NONE | | | |
| EP 4357394 | A1 | | 24-04-2024 | CN | 117321119 | A | 29-12-2023 |
| | | | | EP | 4357394 | A1 | 24-04-2024 |
| | | | | JP | 7331295 | B2 | 22-08-2023 |
| | | | | JP | 2023157922 | A | 26-10-2023 |
| | | | | JP | WO2022264692 | A1 | 22-12-2022 |
| | | | | KR | 20240021748 | A | 19-02-2024 |
| | | | | TW | 202306562 | A | 16-02-2023 |
| | | | | US | 2024261207 | A1 | 08-08-2024 |
| | | | | WO | 2022264692 | A1 | 22-12-2022 |
| JP 2006151992 | A | | 15-06-2006 | JP | 4524254 | B2 | 11-08-2010 |
| | | | | JP | 2006151992 | A | 15-06-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H5200286 A
- JP H11181147 B
- JP 2003252902 A

- JP H11181147 A
- JP 2024078097 A

**Non-patent literature cited in the description**

- Low substituted hydroxypropyl cellulose. Japanese Pharmacopoeia